(19) Europäisches Patentamt
European Patent Office
Office européen des brevets

(11) Veröffentlichungsnummer: **0 403 920 A2**

# (12) EUROPÄISCHE PATENTANMELDUNG

(21) Anmeldenummer: **90111035.3**

(22) Anmeldetag: **12.06.90**

(51) Int. Cl.5: **C08G 59/06, C08G 18/00**

(30) Priorität: **22.06.89 DE 3920410**

(43) Veröffentlichungstag der Anmeldung:
**27.12.90 Patentblatt 90/52**

(84) Benannte Vertragsstaaten:
**BE CH DE ES FR GB IT LI NL**

(71) Anmelder: **BAYER AG**

**D-5090 Leverkusen 1 Bayerwerk(DE)**

(72) Erfinder: **Hess, Bernhard, Dr.**
**Kaldenhausener Strasse 84**
**D-4130 Moers 2(DE)**
Erfinder: **Freitag, Dieter, Dr.**
**Hasenheide 10**
**D-4150 Krefeld 1(DE)**
Erfinder: **Idel, Karsten-Josef, Dr.**
**Am Schwarzkamp 38**
**D-4150 Krefeld 1(DE)**

## (54) Oligomere Epoxidharze auf Basis von speziellen Diphenolen sowie deren Umsetzungsprodukte mit (Meth)acrylsäure und Diisocyanaten.

(57) Die Erfindung betrifft oligomere Epoxidharze, die sich von speziellen Diphenolen bzw. von Diglycidylethern auf Basis dieser Phenole ableiten, Oxazolidinongruppen enthaltende oligomere Epoxidharze auf Basis der neuen Diglycidylether sowie härtbare Mischungen enthaltend die oligomeren neuen Epoxidharze, sowie die Umsetzungsprodukte der neuen oligomeren Epoxidharze mit ungesättigten Carbonsäuren, vorzugsweise in Gegenwart eines Vinylmonomeren zu Phenacrylatharzen, sowie Oxazolidinongruppen enthaltende Phenacrylatharze auf Basis der neuen Diglycidylether.

EP 0 403 920 A2

Xerox Copy Centre

# Oligomere Epoxidharze auf Basis von speziellen Diphenolen sowie deren Umsetzungsprodukte mit (Meth)acrylsäure und Diisocyanaten

Die Erfindung betrifft oligomere Epoxidharze, die sich von speziellen Diphenolen bzw. von Diglycidylethern auf Basis dieser Phenole ableiten, Oxazolidinongruppen enthaltende oligomere Epoxidharze auf Basis der neuen Diglycidylether sowie härtbare Mischungen enthaltend die oligomeren neuen Epoxidharze, sowie die Umsetzungsproukt der neuen oligomeren Epoxidharze mit ungesättigten Carbonsäuren, vorzugsweise in Gegenwart eines Vinylmonomeren zu Phenacrylatharzen, sowie Oxazolidinongruppen enthaltende Phenacrylatharze auf Basis der neuen Diglycidylether.

Cyclohex-1-ylmethylendiphenole sind z.B. aus der GB-A 1 024 012 bekannt. Bicyclo[2,2,1]hept-1-ylmethylendiphenole aus der GB-A 1 024 013. Diese Verbindungen werden beispielsweise als Monomerkomponenten bei der Polycarbonatherstellung eingesetzt z.B. (GB-A 1 024 011). Die Herstellung von Glycidylethern von Hydroxyaryl-3,4-epoxycyclohexylmethanen ist z.B. aus Ref. Zh. Khim., 1985, Abstr. Nr. 19S419 bekannt. Die dort hergestellten Verbindungen sind niedermolekular und besitzen demzufolge eine relativ niedrige Viskosität. Für gewisse Anwendungen, z.B. Laminate, sind hoher aufgebaute Oligomere von Vorteil.

Solche sind z.B. beschrieben in der EP-A 278 900 auf Basis von Cyclohex-1-ylmethylen-diphenolen oder Bicyclo(2.2.1)hept-1-ylmethylendiphenolderivaten. Produkte dieser Art sind jedoch durch die zwischen den Phenolgruppierungen vorhandenen CH-Gruppen gegenüber oxidativen Einflüssen (Bewitterung) und thermische Belastungen empfindlich.

Es wurden neue oligomere Epoxidharze gefunden, die gegenüber oxidativen Einflüssen unempfindlich sind, ein gutes Fließverhalten aufweisen, im gehärteten Zustand hohe Glastemperaturen besitzen und mit (Meth)acrylsäure zu Phenacrylatharzen umgesetzt werden können, die sich durch eine sehr gute Styrolverträglichkeit auszeichnen auch in Form von Oxazolidinongruppen enthaltenden Phenacrylatharzen.

Die Erfindung betrifft Verbindungen der Formel (I) und (II)

(I)

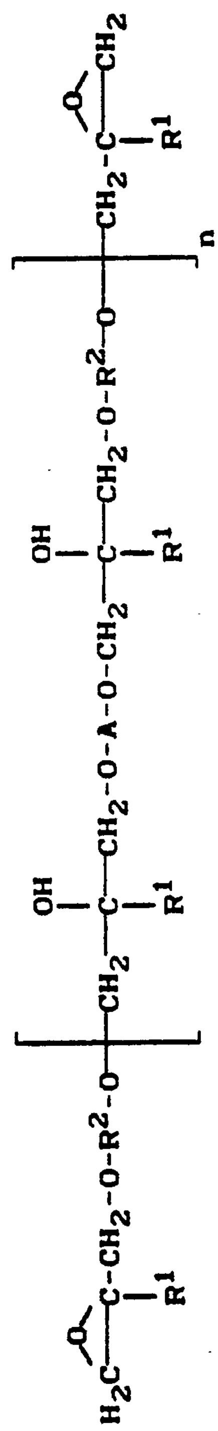

(II)

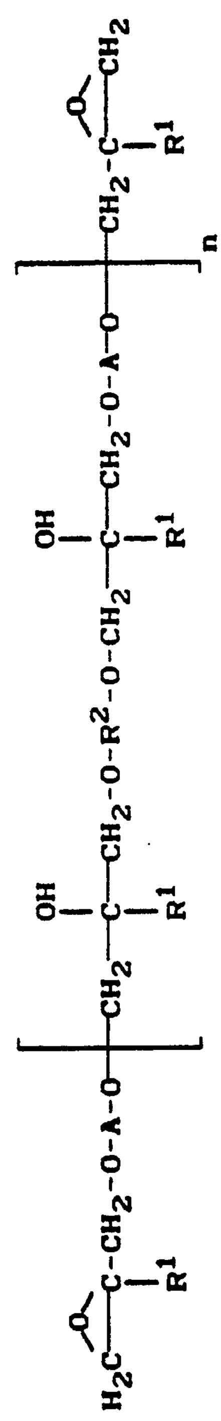

in welchen

$R^1$ für Wasserstoff, $C_1$-$C_{10}$-Alkyl, bevorzugt H, $CH_3$ steht,

$R^2$ für $C_1$-$C_{20}$-aliphatische, $C_5$-$C_{14}$-cycloaliphatische, $C_6$-$C_{24}$-aromatische oder $C_7$-$C_{30}$-araliphatische zweiwertige Reste steht,

n für eine Zahl von 1 bis 20, bevorzugt 1 bis 10, besonders bevorzugt für die Zahl 1, 2, 3, 4 oder 5 steht und

A für einen Rest der Formel (III) steht

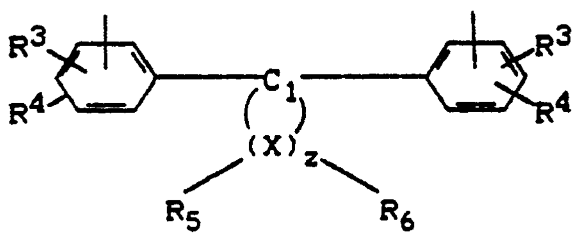

(III),

in welcher

$R^3$ und $R^4$ unabhängig voneinander Wasserstoff, Halogen, bevorzugt Chlor, Brom, $C_1$-$C_8$-Alkyl, $C_5$-$C_6$-Cycloalkyl, $C_6$-$C_{10}$-Aryl, bevorzugt Phenyl und $C_7$-$C_{12}$-Aralkyl, bevorzugt Phenyl-$C_1$-$C_4$-Alkyl, insbesondere Benzyl bedeuten,

z für die Zahl 4, 5, 6 oder 7, bevorzugt 4 oder 5 steht,

$R^5$ und $R^6$ für jedes X individuell wählbar sind und unabhängig voneinander für Wasserstoff, $C_1$-$C_{12}$-Alkyl, bevorzugt ist Methyl, Ethyl stehen und

X für Kohlenstoff steht.

Besonders bevorzugt ist X mit der Maßgabe, daß an mindestens einem Atom X, vorzugsweise an 1 oder 2 Atomen X, insbesondere an 1 Atom X, $R^5$ und $R^6$ gleichzeitig $C_1$-$C_{12}$-Alkyl, vorzugsweise Methyl bedeuten, wobei mindestens ein X-Atom $\alpha$-ständig zu C1 nicht geminal dialkyl-substituiert ist, vorzugsweise beide X-Atome $\alpha$-ständig zu C1 nicht geminal dialkylsubstituiert sind und insbesondere das X-Atom, welches geminal dialkylsubstituiert ist, in $\beta$-Stellung zu C1 steht.

Die freien Bindungen sollen vorzugsweise in 3- oder 4-Position zur Brücke C1 stehen.

Besonders bevorzugte Beispiele in Formel (I) und (II) enthalten als Gruppe A Reste der Formeln (IV), (V) und/oder (VI)

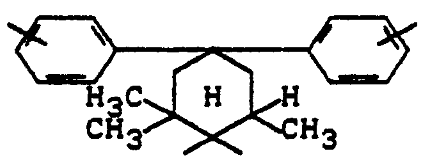

(IV)

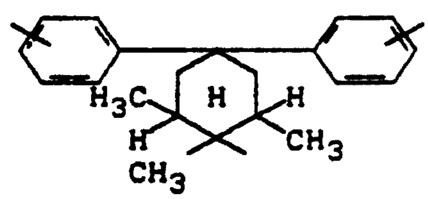

(V)

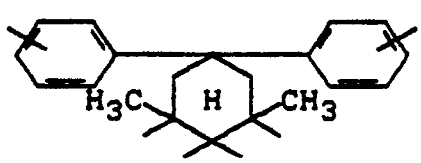

(VI)

Sie sind in Form der Diole durch Kondensation von hydriertem Isophoron oder der entsprechenden di- oder trialkylierten Ccylohexanone mit Phenol in verschiedenen Isomeren erhältlich. Ganz besonders bevorzugt sind die Beispiele, die sich vom hydrierten Isophoron ableiten.

Leitet sich $R^2$ von einem aliphatischen Diol ab, so handelt es sich dabei um geradkettige oder verzweigtkettige Alkylenreste, die gegebenenfalls durch Sauerstoff- oder Schwefelatome unterbrochen sein können, und die gegebenenfalls Substituenten (z.B. Halogen wie Cl, Br) tragen können.

Bevorzugt werden unsubstituierte geradkettige $C_2$-$C_{20}$-Alkylenreste, z.B. Ethylen, Tri-, Tetra-, Penta-, Hexa-, Hapta-, Octa-, Deca-, Dodeca-, Tetradeca-, Hexadeca-, Octadeca- Eicosamethylen, besonders bevorzugt wird Tetramethylen.

Das $R^2$ zugrundeliegende Diol kann auch ein Poly-(oxyalkylen)-glykol oder ein Poly-(thioalkylen)-glykol sein. Bevorzugt sind die sauerstoffhaltigen Derivate, z.B. Poly-(ethylen)-glykol, Poly-(propylen)-glykol, Poly-(butylen)-glykol mit 2-60 Monomereinheiten.

Leitet sich $R^2$ von einem cycloaliphatischen Diol ab, so handelt es sich dabei beispielsweise um ein Diol mit einem cycloaliphatischen Ring mit 5-7 Kohlenstoffatomen, der gegebenenfalls Teil einer aliphatischen Kette sein kann oder der gegebenenfalls Substituenten direkt am Ring trägt. Beispiele für solche Reste sind vorzugsweise Cyclopentylen, Cyclohexylen oder Cycloheptylen. Besonders bevorzugt wird Cyclohexylen, insbesondere das 1,3-oder das 1,4-Cyclohexylen sowie Hexahydroxylylen.

Basiert $R^2$ auf einem aromatischen Diol, so leitet sich dieser Rest bevorzugt von einem ein- oder zweikernigen Phenol ab. Beispiele dafür sind 1,2-Phenylen oder insbesondere 1,3- oder 1,4-Phenylen sowie Reste der Formel (VII)

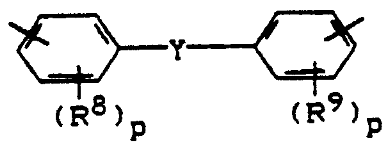

(VII),

worin

die freien Bindungen vorzugsweise in 3- oder 4-Position zur Brücke Y stehen und

Y eine direkte C-C-Bindung ist oder $-CH_2-$, $-CHCH_3-$, $-C(CH_3)_2-$, -O-, -S-, $-SO_2-$, oder -CO- ist,

p eine ganze Zahl von 0 bis 4, bevorzugt 0, 1 oder 2 ist und

$R^8$ und $R^9$ unabhängig voneinander $C_1$ bis $C_6$ Alkyl, Chlor oder Brom bedeuten.

Bevorzugte Reste $R^2$ sind Diphenylmethan-4,4′-diyl, Diphenylether-4,4′-diyl, Diphenylsulfon-4,4′-diyl und ganz besonders Diphenyl-2,2-propyliden-4,4′-diyl. Als araliphatischer Rest ist $R^2$ beispielsweise Xylylen.

Die Verbindungen der Formel (I) oder (II) liegen in der Regel als Gemisch von Komponenten unterschiedlicher Kettenlänge vor. Das mittlere Molekulargewicht (Zahlenmittel; ermittelt durch Gel-Permeationschromatographie) ist mindestens so groß wie das Molekulargewicht der entsprechenden reinen monomeren Verbindung (n = 1). Vorzugsweise ist der Mittelwert von n eine Zahl von 1 bis 10 und ganz besonders von 1 bis 5.

Die Herstellung der oligomeren Epoxide gemäß den Formeln I oder II wird in Analogie zu bekannten Verfahren durchgeführt (z.B. die Synthese langkettiger Diglycidylether auf Bisphenol-Basis z.B. analog "Epoxy-Handbook" Kap. 2 bis 9) von Lee und Neville.

Die vorliegende Erfindung betrifft auch Umsetzungsprodukte der Verbindungen gemäß Formel (II) mit n = 0 mit Diisocyanaten zu Oxazolidinongruppen enthaltenden Oligoepoxidharzen gemäß DE-A 3 720 759.

Als Diisocyanate sind bevorzugt geeignet aromatische Diisocyanate, die zwei unterschiedlich reaktive NCO-Gruppen in einer Menge von mindestens 1/4 des Gewichts des Diisocyanats aufweisen, wie z.B. o,p-Toluylendiisocyanat, Diphenylmethan-2,4′-diisocyanat, Naphthylendiisocyanat-1,3 allein oder im Gemisch untereinander oder im Gemisch mit anderen Diisocyanaten, die zwei gleichreaktive NCO-Gruppen aufweisen, o,o-Toluylendiisocyanat, Naphthylendiisocyanat-1,5. In diesem Falle sollen die Diisocyanate mit unterschiedlich reaktiven NCO-Gruppen mindestens 1/4 des Gesamtgewichts der Diisocyanate ausmachen.

Die für das erfindungsgemäße Verfahren geeigneten Katalysatoren sind Phosphoniumsalze.

Das Verfahren wird bei einer Temperatur von 140 bis 180°C, bevorzugt von 150 bis 170°C durchgeführt.

Das Mengenverhältnis, in dem die Bisepoxide mit den Diisocyanaten umgesetzt werden, liegt bei 1,4 bis 2,5 Epoxidgruppen pro einer NCO-Gruppe, vorzugsweise 1,6 bis 2,2 Epoxidgruppen pro einer NCO-Gruppe. In jedem Fall bleiben reaktive Epoxidgruppen im Endprodukt übrig, die für Folgereaktionen oder zur Vernetzung zur Verfügung stehen. Je geringer der Überschuß an Epoxidgruppen gegenüber den NCO-Gruppen ist, um so höher wird das Molekulargewicht der modifizierten Epoxidharze und umso höher deren Schmelzviskosität.

Die Erfindung betrifft auch ein härtbares Gemisch enthaltend

a) mindestens eine Verbindung der Formel I, II oder Oligoepoxidharze mit Oxazolidinongruppen,

b) ein für die Härtung besagten Gemisches ausreichende Menge eines Epoxidharzhärters und

c) gegebenenfalls einen Härtungsbeschleuniger.

Als Epoxidhärtungsmittel b) kommen saure, basische oder katalytische Härter in Frage. Dazu zählen beispielsweise Amine, Amide, wie aliphatische, cycloaliphatische oder aromatische, primäre, sekundäre oder tertiäre Amine, beispielsweise Hexamethylendiamin, N,N-Diethylpropylendiamin, Bis-(4-aminocyclohexyl)-methan, 3,5,5-Trimethyl-3-(aminomethyl)-cyclohexylamin ("Isophorondiamin", 2,4,6-Tris-(dimethylaminomethyl)-phenol, p-Phenylendiamin, Bis-(4-aminophenyl)-methan; Polyamide, beispielsweise solche aus aliphatischen Polyaminen und di-oder trimerisierten ungesättigten Fettsäuren; mehrwertige

Phenole, wie beispielsweise Resorcin, 2,2-Bis(4-hydroxyphenyl)-propan, Phenol-Formaldehydharze (Phenol-Novolake); Bortrifluorid und seine Komplexe mit organischen Verbindungen, beispielsweise $BF_3$-Ether-Komplexe, $BF_3$-Amin-Komplexe; mehrbasische Carbonsäuren und deren Anhydride, beispielsweise Phthalsäureanhydrid, Tetrahydrophthalsäureanhydrid, Hexahydrophthalsäureanhydrid oder die entsprechenden Säuren u.s.w.

Man kann bei der Härtung außerdem Härtungsbeschleuniger c) einsetzen; solche Beschleuniger sind z.B. tertiäre Amine, deren Salze oder quaternäre Ammoniumverbindungen, z.B. Benzyldimethylamin, 2,4,6-Tris(dimethylaminomethyl)phenol, 1-Methylimidazol, 2-Ethyl-4-methylimidazol, 4-Aminopyridin, 2,4,6-Tripentylammoniumphenolat, Tetramethylammoniumchlorid; Alkalimetallalkoholate, z.B. Na-Alkoholate von 2,4-Dihdyroxy-3-hydroxymethylpentan.

Derartige härtbare Gemische können geeignete Weichmacher wie Dibutylphthalat, Dioctylphthalat oder Trikresylphosphat, reaktive Verdünner wie Phenyl-oder Kresylglycidylether, Butandiolglycidylether, Hexahydrophthalsäurediglycidylester, usw. enthalten.

Die härtbaren Gemische können vor der Härtung in irgendeiner Phase mit Streck-, Füll und Verstärkungsmitteln wie Steinkohlenteer, Bitumen, Textilfasern, Glasfasern, Absbestfasern, Borfasern, Kohlenstoff-Fasern, mineralischen Silikaten, Glimmer, Quarzmehl, Aluminiumoxidhydrat, Bentoniten, Kaolin, Kieselsäureaerogel, Metallpulvern, z.B. Aluminiumpulver, Eisenpulver, mit Pigmenten, Farbstoffen wie Russ, Oxidfarben, Titandioxid, u.a. versetzt werden. Man kann den härtbaren Gemischen auch andere übliche Zusätze, z.B. Flammschutzmittel, wie Antimontrioxid, Thixotropiermittel, Verlaufmittel ("flow control agents") wie Silicone, Wachse oder Stearate (welche zum Teil auch als Formtrennmittel Anwendung finden), zusetzen.

Die Herstellung der erfindungsgemäßen härtbaren Mischungen kann in üblicher Weise mit Hilfe bekannter Mischaggregate (Rührer, Kneter, Walzen etc.) erfolgen.

Die erfindungsgemäßen härtbaren Epoxidharzmischungen finden ihren Einsatz vor allem auf den Gebieten des Oberflächenschutzes, der Elektrotechnik, der Laminierverfahren und im Bauwesen. Sie können in jeweils dem speziellen Anwendungszweck angepaßter Formulierung, im ungefüllten oder gefüllten Zustand, als Anstrichmittel, Lacke, wie Sinterpulverlacke, als Pressmassen, Tauchharze, Giesharze, Spritzgußformulierungen, Imprägnierharze und Klebmittel, als Werkzeugharze, Laminierharze, Dichtungs- und Spachtelmassen, Bodenbelagmassen und Bindemittel für mineralische Aggregate verwendet werden.

Bevorzugt sind sie als Sinterpulverlacke, Imprägnier-und Laminierharze, insbesondere als Imprägnier- und Laminierharze, verwendbar.

Die Härtung der erfindungsgemäßen glycidylgruppenhaltigen Copolymeren wird zweckämßig im Temperaturintervall von 50°C bis 300°C, bevorzugt von 80 bis 250°C, durchgeführt.

Man kann die Härtung in bekannter Weise auch zwei- oder mehrstufig durchführen, wobei die erste Härtungsstufe bei niedriger Temperatur und die Nachhärtung bei höherer Temperatur durchgeführt wird.

Die Härtung kann gewünschtenfalls auch derart in 2 Stufen erfolgen, daß die Härtungsreaktion zunächst vorzeitig abgebrochen bzw. die erste Stufe bei wenig erhöhter Temperatur durchgegührt wird, wobei ein noch schmelzbares und/oder lösilches, härtbares Vorkondensat (sogenannte "B-Stufe") aus der Epoxi-Komponente a), dem Härter b) und dem gegebenenfalls anwesenden Beschleuniger c) erhalten wird. Ein derartiges Vorkondensat kann z.B. zur Herstellung von "Prepregs", Pressmassen oder Sinterpulvern dienen.

Die vorliegende Erfindung betrifft auch Umsetzungsprodukte der Verbindungen gemäß der Formeln (I) und (II) - Formel (II) auch mit $n = O$ - sowie Umsetzungsprodukte der beschriebenen Oxazolidinongruppen enthaltenden Oligoepoxidharze mit ungesättigten Carbonsäuren, bevorzugt Acryl- oder Methacrylsäure in Gegenwart eines Katalysators, vorzugsweise in Gegenwart eines Vinylmonmeren gemäß DE-A 3 723 196.

Als ungesättigte Carbonsäuren sind ethylenisch ungesättigte Carbonsäuren, z.B. Methacrylsäure, Acrylsäure, Zimtsäure sowie Halbester aus einer Dicarbonsäure und einem ungesättigten Hydroxyalkylcarbonsäureester geeignet. Bevorzugt ist Acrylsäure und Methacrylsäure.

Die Epoxidharze und ungesättigten Carbonsäuren werden bevorzugt in etwa stöchiometrischen Verhältnissen eingesetzt, d.h. pro Epoxidäquivalent des Harzes wird etwa ein Äquivlent Carbonsäuren eingesetzt.

Als Katalysatoren sind geeignet quarternäre Ammoniumsalze, wie Halogenide, Acetate oder Formiate.

Solche Katalyatoren werden z.B. in der GB-A 1 364 804 beschrieben. Bevorzugt werden Tetraethyl- und Tetrabutyl ammonium-bromid oder -chlorid und Triethylbenzylammonium-chlorid oder -bromid.

Als Katalysatoren sind geeignet Phosphoniumhalogenide.

Geeignet Phosphoniumsalze sind z.B. in der EP-A 99 334 beschrieben. Beispiele für besonders bevorzugte Phosphoniumverbindungen sind Tetrabutylphosphonium-bromid oder -chlorid oder Triphenylbenzylphosphonium-chlorid oder -bromid.

Als Vinylmonomere können solche eingesetzt werden, die gegenüber Epoxidgruppen unter den Umsetzungsbedingungen inert sind, z.B. Styrol, kernchlorierte und -alkylierte bzw. -alkenylierte Styrole, wobei die Alkylgruppen 1 bis 4 C-Atome enthalten können wie Vinyltoluol, Divinylbenzol, α-Methylstyrol,

tert.-Butylstyrol, Chlorstyrole, Vinylester von Carbonsäuren mit 2 bis 6 C-Atomen, bevorzugt Vinylacetat, Vinylpyridin, Vinylnaphthalin, Vinylcyclohexan, Acryl- und Methacrylsäureester ohne funktionelle Gruppen, Allylverbindungen wie Allylbenzol und Allylester wie Allylacetat, Phthalsäurediallylester, Isophthalsäurediallylester, Allylcarbonate, Triallylphosphonat, Triallylcyanurat. Bevorzugt ist Styrol.

Die beschriebenen Phenacrylatharze können auch durch Aufsäuerung mit Säureanhydriden zu sauren Halbestern mit sauren Gruppen ausgerüstet werden. Über diese können die Harze dann z.B. mit MgO eingedickt werden (z.B. bei der Verarbeitung zu sogenannten Harzmatten.

Zur Aufsäuerung (Umsetzung mit Säureanhydriden zu sauren Halbestern) der gebildeten Phenacrylatharzlösung in Vinylmonomeren geeignete Säureanyhdride sind vorzugsweise die Anyhdride der Bernsteinsäure, Phthalsäure, Tetrahydrophthalsäure, Methyltetrahydrophthalsäure, Hexahydrophthalsäure, Methylhexahyrophthalsäure, Endomethylentetra- oder -hexahydrophthalsäure, Hexachlorendomethylentetrahydrophthalsäure, usw.; bevorzugt werden die Anhydride der (Methyl)Tetra- oder (Methyl)Hexahydrophthalsäure.

Die Menge an Säureanhydrid soll so bemessen sein, daß sich für das aufgesäuerte Phenacrylatharz eine Säurezahl von 15 bis 80, bevorzugt 30 bis 60 bestimmen (z.B. errechnen oder titrieren) läßt.

Die Erfindung betrifft weiterhin die Verwendung der härtbaren Massen, insbesondere der Acrylat- oder Methacrylatgruppen enthaltenden Umsetzungsprodukte der Verbindungen der Formeln I, II oder der Oxazolidinongruppen enthaltenden Oligoepoxidharze bevorzugt in Vinylmonomerenlösung, wie Styrol, alkylierte oder halogenierte Styrole zur Herstellung von Beschichtungen, Gießharzen, Spachtelmassen, Preßmassen, faserhaltigen Laminaten und Klebstoffen.

Beispiele

## Kennzeichnung der Abkürzungen:

| | | |
|---|---|---|
| X20 | : | Bisphenol-A-bisglycidylether, technisch, Epoxidequivalentgewicht: 186 |
| BPA | : | Bisphenol A |
| HIP | : | Bis(4-hydroxyphenyl)3,3,5-trimethylcyclohexan)[1] |
| HIP-(EP) | : | Bis(4-hydroxyphenyl)-3,3,5-trimethylcyclohexan(bisglycidylether); Epoxidequivalentgewicht: 235 |
| MDI-M | : | Gemisch aus 59 % Diphenylmethandiisocyanat-4,4', 34 % -2,4' und 6 % -2,2' |
| Cu-Lösung | : | Kupfernaphthenat, 10 %ige Lösung in Styrol, enthaltend 1 % Kupfer-Metall. |

Katalysator A: Tetrabutylphosphoniumbromid

Katalysator B: Triethylbenzylammoniumchlorid

1)

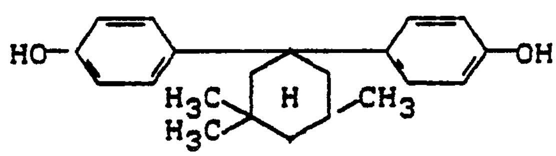

Die in den Tabellen angegebenen Mengen sind Gewichtsteile.

Herstellung der Beispiele 1 und 2 und Vergleichsbeispiele:

Es werden die in Tabelle I aufgeführten Epoxidharzmengen und der Katalysator B in einen Rührkolben eingewogen und unter Stickstoff auf die in der Tabelle I angegebene Reaktionstemperatur geheizt. Bei dieser Temperatur wird unter Rühren portionsweise in ca. 30 min. HIP oder BPA zugegeben und die Temperatur 3 Stunden gehalten. Nach dieser Zeit ist der Epoxidwert auf den berechneten Wert abgesunken und kein phenolisches OH mehr vorhanden. Die Epoxidequivalentgewichte und der Schmelzbereich der Oligoepoxidharze sind ebenfalls tabellarisch aufgeführt. Ferner ist in Tabelle I die Glastemperatur der gehärteten Produkte angegeben nach der üblichen Heißhärtung mit Hexahydrophthalsäureanhydrid entsprechend den Epoxidequivalentgewichten.

Die Tabelle zeigt deutlich die höheren Glastemperaturen der Beispiele gegenüber den Vergleichsbeispielen.

Tabelle I:

| Beispiel: | 1 | | 2 | | |
|---|---|---|---|---|---|
| Vergleichsbeispiel | | 1a | | 2a | 1b |
| X20 | 100 | 100 | - | - | 100 |
| HIP-Ep | - | - | 100 | 100 | - |
| HIP | 41,0 | - | 32,6 | - | - |
| BPA | - | - | - | - | 30 |
| Katalysator B | 0,05 | - | 0,05 | - | 0,05 |
| Reaktionstemperatur (° C) | 150 | - | 185 | - | 130 |
| Epoxidequivalentgewicht | 514 | 186 | 675 | 232 | 500 |
| Schmelzbereich [° C] | 90 | flüssig | 130 | 40/50 | 70 |
| Glastemperatur (° C) | 200 | 125 | 218 | 150 | 115 |

Die in den Tabelle angegebenen Mengen sind Gewichts teile.

Beispiel 3 und Vergleichsbeispiel 3a

(Oxazolidinongruppen enthaltendes Oligoepoxidharz)

Die in Tabelle II jeweils aufgeführten Mengen an Bis-epoxidharzen und Katalysator A werden in einen Kolben mit Rührer und Gaseinleitungsrohr eingewogen und unter Stickstoff auf 160° C aufgeheizt. Zu der Schmelze wird das Diisocyanat (MDI-M) so schnell zugetropft, daß eine Temperatur von 170° C nicht überschritten wird, was durch Abschalten der Heizung oder durch Kühlung gesteuert werden kann. Nach der Zugabe des Diiscoyanats wird bei 160° C nachgerührt bis der berechnete Epoxidwert erreicht und kein reaktives NCO mehr nachweisbar ist. Die fertige Schmelze wird heiß abgefüllt und erstarren gelassen.

Tabelle II:

| Beispiel/Vergleich: | 3 | 3a |
|---|---|---|
| X20 | - | 224,5 |
| HIP-EP | 289 | - |
| Katalysator A | 0,74 | 0,22 |
| MDI-M | 80 | 75,5 |
| Reaktionszeit (Std.) | 1 | 3 |
| Equival. Epoxid: NCO | 2:1 | 2:1 |
| % NCO | 0 | 0 |
| Epoxidequiv. gew.: | 595 | 503 |
| Schmelzbereich (° C) | 134/140 | 88/94 |

Heißhärtung der Beispiele 3 und 3a in Kombination mit einem flüssigen Bisphenol-A-bisglycidylether:

(Tabelle III)

| Zusammensetzung der Mischungen: | | |
|---|---|---|
| Bisphenol-A-bisglycidylether, technisch | | 75 Gew.-Tl. |
| Oxazolidinonepoxidharz Beispiel 3 u. 3a | | 25 Gew.-Tl. |
| Hexahydrophthalsäureanhydrid | | 70 Gew.-Tl. |
| Dimethylbenzylamin | | 1,7 Gew.-Tl. |
| Glastemperatur mit Beispiel: | 3 | 3a |
| | 145° | 110°C |

Jeweils 25 g Oxazolidinonepoxidharz gemäß Beispiel 3 oder 3a werden in 75 g Bisphenol-A-bisglycidylether, technische Qualität, bei 100 bis 110°C gelöst und zu der Lösung 70 g Hexahydrophthalsäureanhydrid zugegeben. Die Schmelze wird auf Raumtemperatur abgekühlt, 1,7 g Dimethylbenzylamin eingerührt, evakuiert und danach zwischen zwei Glasplatten mit einem Abstand von 4 mm gegossen. Die Platten werden 4 Stunden bei 80°C angehärtet, danach 16 Stunden bei 120°C ausgehärtet und danach entformt. Das gehärtete Material wird in Normstäbe geschnitten und mechanisch vermessen. Es zeigt die in Tabelle III aufgeführten Werte.

Beispiel 3 zeigt im Vergleich zu Beispiel 3a deutlich höhere Wärmeformbeständigkeit.

Die folgenden Beispiele und Vergleichsbeispiele beschreiben Umsetzungsprodukte von Oligoepoxidharzen mit Methacrylsäure in Vinylmonomerenlösung.

Herstellung der Beispiele 4 bis 7 und Vergleichsbeispiel:

Die in der Tabelle IV jeweils aufgeführten Gew.-Tl. der Beispiele 1 bis 3, deren Zusammensetzung zur besseren Übersicht vorab tabellarisch aufgerührt ist, werden in einen Kolben mit Rührer und Gaseinleitungsrohr eingewogen und in Styrol und gegebenenfalls Vinyltoluol, das die aufgeführten Mengen an Inhibitoren und Cu-Lösung enthält, gelöst. Zu der Lösung wird die entsprechende Katalysatormenge B zugegeben und unter Rühren und Luftdurchleiten auf 110°C erwärmt. Innerhalb von ca. 15 Min. wird die Methacrylsäure zugetropft und die Lösung bei 110°C gehalten bis der Methacrylsäuregehalt auf < 1 % bezogen auf sämtliche Komponenten ohne Styrol, gesunken ist.

Die Beispiele und Vergleichsbeispiele der Tabelle IV zeigen nach Heißhärtung mit 1 Gew.-% Tert.-butylperbenzoat bei 120°C und anschließende Temperung über 15 Stunden bei 120°C die in der Tabelle aufgerühte Wärmeformbeständigkeit. Sie liegt deutlich höher als die des Vergleichsbeispiels.

Tabelle IV:

| Beispiel: | 4a | 4 | 5 | 6 | 7 |
|---|---|---|---|---|---|
| X20 | 100 | 100 | - | - | - |
| HIP-EP | - | - | 100 | 100 | 100 |
| BPA | 30 | - | - | - | - |
| HIP | - | 41 | 32,6 | - | - |
| MDI-M | - | - | - | 27,7 | - |
| Beispielharz | 1b | 1 | 2 | 3 | - |
| Katalysator B | 0,75 | 0,75 | 0,75 | 0,75 | 0,75 |
| Styrol | 113,3 | 134 | 123,3 | 56,6 | 88,9 |
| Vinyltoluol | - | - | - | 22,7 | - |
| Toluhydrochinon | 0,038 | 0,038 | 0,038 | 0,038 | 0,038 |
| Di-tert.-butylchinon | 0,038 | 0,038 | 0,038 | 0,038 | 0,038 |
| Cu-Lösung | 0,38 | 0,38 | 0,38 | 0,38 | 0,38 |
| Methacrylsäure | 22 | 22,8 | 18,1 | 20,4 | 33,4 |
| Viskosität bei 20 °C (mPas) | 1250 | 710 | 1000 | 31.400 | 117 |
| Martensgrad | 100 | 107 | 116 | 125 | 134 |
| Glastemperatur (°C) | 119 | 125 | 129 | 136 | 150 |

## Ansprüche

1. Verbindungen der Formeln (I) und (II)

(I)

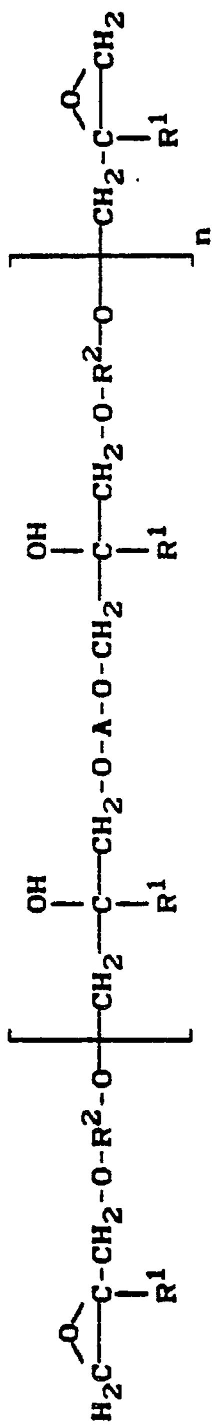

(II)

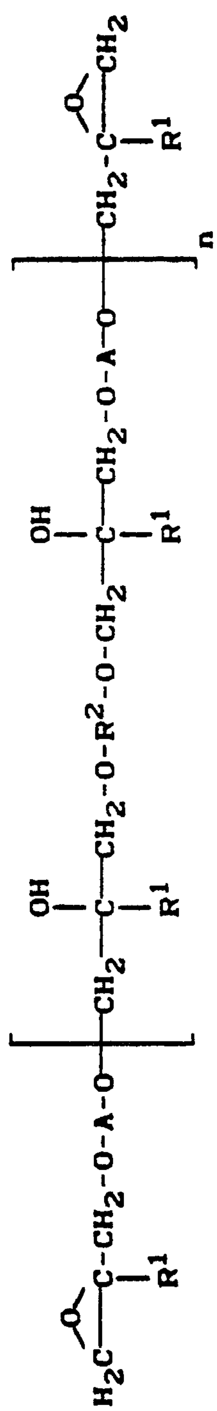

in welchen

$R^1$ für Wasserstoff, $C_1$-$C_{10}$-Alkyl, bevorzugt H, $CH_3$ steht,

$R^2$ für $C_1$-$C_{20}$-aliphatische, $C_5$-$C_{14}$-cycloaliphatische, $C_6$-$C_{24}$-aromatische oder $C_7$-$C_{30}$-araliphatische zweiwertige Reste steht,

n für eine Zahl von 1 bis 20, bevorzugt 1 bis 10, besonders bevorzugt für die Zahl 1, 2, 3, 4 oder 5 steht und

A für einen Rest der Formel (III)

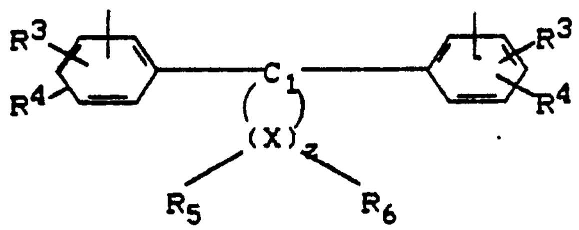

(III),

in welcher
$R^3$ und $R^4$ unabhängig voneinander Wasserstoff, Halogen bevorzugt Chlor, Brom, $C_1$-$C_8$-Alkyl, $C_5$-$C_6$-Cycloalkyl, $C_6$-$C_{10}$-Aryl, bevorzugt Phenyl und $C_7$-$C_{12}$-Aralkyl, bevorzugt Phenyl-$C_1$-$C_4$-Alkyl, insbesondere Benzyl bedeuten,
z für die Zahl 4, 5, 6 oder 7, bevorzugt 4 oder 5 steht,
$R^5$ und $R^6$ für jedes X individuell wählbar sind und unabhängig voneinander für Wasserstoff, $C_1$-$C_{12}$-Alkyl, bevorzugt Methyl, Ethyl stehen und
X für Kohlenstoff steht.

2. Härtbares Gemisch enthaltend
   a) mindesens eine Verbindung gemäß Anspruch 1
   b) eine für die Härtung des Gemisches ausreichende Menge eines Epoxidharzhärters und
   c) gegebenenfalls einen Härtungsbeschleuniger.

3. Umsetzungsprodukte von Verbindungen gemäß Anspruch 1 mit Diisocyanaten zu Oxaolidongruppen enthaltenden Oligoepoxidharzen.

4. Härtbares Gemisch, enthaltend
   a) mindestens eine Verbindung der Formel I, II oder Oligoepoxidharze mit Oxazolidinongruppen,
   b) ein für die Härtung besagten Gemisches ausreichende Menge eines Epoxidharzhärters und
   c) gegebenenfalls einen Härtungsbeschleuniger.

5. Verwendung der härtbaren Massen gemäß Ansprüchen 1 und 4 für die Herstellung von Beschichtungen, Gießharzen, Spachtelmassen, Preßmassen, faserhaltigen Laminaten und Klebstoffen.